# EUROPEAN PATENT SPECIFICATION

(11) **EP 1 558 219 B1**
(45) Date of publication and mention of the grant of the patent: **03.10.2007**
(21) Application number: 03751723.2
(22) Date of filing: 16.10.2003
(51) Int. Cl.: A61K 9/20, A61K 31/551, A61P 25/18

(54) **PHARMACEUTICAL FORMULATION OF OLANZAPINE**
PHARMAZEUTISCHE FORMULIERUNG ENTHALTEND OLANZAPIN
FORMULATION PHARMACEUTIQUE D'OLANZAPINE

(30) Priority: 18.10.2002 SI 200200255
(43) Date of publication of application: 03.08.2005
(73) Proprietor: KRKA, tovarna zdravil, d.d., Novo mesto, 8501 Novo mesto (SI)
(72) Inventor: PERC, Stanka, 8344 Vinica pri Crnomlju (SI); BANKO, Ivanka, 8361 Dvor (SI); KOLENC, Ivanka, 8000 Novo mesto (SI)
(74) Representative: UEXKÜLL & STOLBERG
(86) International application number: PCT/SI2003/000036
(87) International publication number: WO 2004/035027

(56) References cited:
- EP-A1- 0 830 858
- WO-A1-03/086361

## Description

The present invention relates to a pharmaceutical composition containing a homogeneous mixture of (a) 2-methyl-4-(4-methyl-1-piperazinyl)-10H-thieno [2,3-b][1,5]benzodiazepine, in the continuation referred to by its generic name of olanzapine, or a pharmaceutically acceptable salt thereof, (b) a monosaccharide and/or oligosaccharide, and (c) a polysaccharide.

### Technical Field

Olanzapine is an antagonist of dopamine at D-1 and D-2 receptors and, in addition, it has antimuscarinic anticholinergic properties and antagonist activities at 5HT-2 receptor sites. It also shows antagonist activity at noradrenergic α-receptors. The compound is a neuroleptic with relaxant, anxiolitic or antiemetic properties, and is useful in treating psychotic conditions such as schizophrenia, schizophreniform disorders and acute mania. At lower doses the compound is indicated for use in the treatment of mild anxiety states.

### Technical Problem

Due to moisture-sensitive, metastable nature of olanzapine, the formulation presently on the market requires several steps in its technological preparation such as granulation, compression, sub-coating and coating in order to assure the protection of the active substance from moisture and light. Such a technological process is technologically and economically demanding, therefore a need exists to develop a stable formulation obtained by a technologically and economically more acceptable process.

According to present invention there is provided a formulation with high stability without any undesired discoloration or poor dose uniformity. The formulations of the present invention can be prepared by a simple technological process such as direct compression.

### Prior Art

In EP 0454436 B1 it is reported that pharmaceutical compositions of olanzapine can be prepared by using conventional techniques. The active ingredient can be mixed with a carrier such as lactose, dextrose, sucrose, sorbitol, mannitol, starches, gum acacia, calcium phosphate, alginates, tragacanth, gelatin, syrup, methyl cellulose, methyl and propyl-hydroxy benzoate, talc, magnesium stearate or mineral oil. In a specific example there is given a formulation prepared by granulation and compressing and containing magnesium stearate, microcrystalline cellulose, povidone and starch. Depending on the method of administration, the compositions may be formulated as tablets, capsules, injection solutions for parenteral use, suspensions or elixirs for oral use or suppositories.

EP 0733367 B1 relates to a stable solid oral formulation comprising olanzapine intimately mixed with a bulking agent, a binder, a disintegrant, a dry binder and a lubricant, whereupon such solid oral formulation is coated with a polymer. The coating with certain polymers is said to provide uniformity and physical stability and to effectively prevent the undesired discoloration phenomenon in the formulation. Ambient conditions, elevated temperatures and moist environment aggravate the problem of discoloration, which is believed to be particularly disturbing when a tablet formulation is administered to a psychotic patient. The process for the preparation of the formulation comprises the steps of wet granulation, drying, blending with additional excipients and compression. The obtained cores are first sub-coated with HPMC in order to avoid a direct contact of the active ingredient with polyethylene glycol and subsequently coated with a coating suspension. In the description it is pointed out that olanzapine may form an undesired crystal form in the presence of certain solvents and excipients, therefore it is desired to prepare the formulation using a method which does not require a dissolution of the olanzapine substance. They believe that a dry blend direct compression process or a dry granulation process for preparing solid oral formulations create a greater chance for poor dose uniformity to occur. In the light of the potent nature of olanzapine, a consistent dose uniformity is imperative therefore they used high-shear aqueous wet granulation with fluid bed drying as the most effective method for preparing pharmaceutically elegant and stable oral olanzapine formulations. Though the presence of solvents can cause undesirable conversions they could not avoid the use of wet granulation. This problem has been successfully solved by the present invention.

EP 0830858 A1 relates to a formulation containing a coated active ingredient. The coating provides a uniform physical stability and effectively prevents the undesired discoloration phenomenon in the formulation. It is stated that the known formulation described in US 5,229,382, a counterpart to EP 0454436, shows the tendency of olanzapine to undesirably discolor. They stated that olanzapine undergoes undesirable discoloration when contacted with certain excipients including powder blends.

WO 98/11897 discloses a formulation of olanzapine in a fixed combination with fluoxetine comprising microcrystalline cellulose, silicone dioxide and stearic acid. The components are blended and compressed to form tablets.

### Description of the Invention

According to the present invention there is disclosed a pharmaceutical formulation, comprising (a) as the active ingredient, 2-methyl-4-(4-methyl-1-piperazinyl)-10H-thieno [2,3-b][1,5]benzodiazepine or a pharmaceutically acceptable salt thereof, (b) a monosaccharide and/or oligosaccharide, and (c) a polysaccharide. Components (b) and (c) serve as diluents for the active ingredient. The formulations of the present invention preferably also contain a binder, a disintegrating agent and a lubricant. The formulations of the present invention are obtainable by homogeneously mixing olanzapine or a pharmaceutically acceptable salt thereof with a monosaccharide and/or oligosaccharide and/or a reduced or oxidised form thereof, a polysaccharide and optionally one or more additional excipients, followed by direct compression of the mixture into tablets in the absence of any solvent. The formulations of the present invention have the form of tablets. The term "tablet" as used herein is intended to encompass compressed pharmaceutical dosage formulations of all shapes and sizes. Uncoated tablets are particularly preferred. According to the present invention the tablets are prepared by direct compression. During our research we have found that the discoloration phenomenon is probably caused by the formation of olanzapine hydrates that have a less intensive colour than olanzapin. In order to prevent the formation of hydrates, the process for the manufacture of the pharmaceutical formulation should be performed without using solvents. It was surprisingly found by the present inventors that stable pharmaceutical formulations comprising olanzapine as the active ingredient, which do not show any undesired discoloration and have an excellent dose uniformity, can be prepared by a simple direct compression process if olanzapine or a pharmaceutically acceptable salt thereof is first homogeneously mixed with certain excipients and then subjected to direct compression. The direct compression is performed in the absence of any solvent. In view of the fact that the excipients used by the present inventors are commonly used for manufacturing tablets, the finding that they allow the production of stable olanzapine formulations without any need for a coating or wet granulation was totally unexpected.

The most obvious advantage of direct compression is economy. Savings can occur in a number of areas including reduced processing time and thus reduced labour costs, fewer manufacturing steps and pieces of equipment, less process validation, and a lower consumption of power.

The most significant advantages concerning the tablet quality are the processing without any need for moisture and heat that are inherent in most wet granulation procedures and the avoidance of high compaction pressures involved in producing tablets by slugging or roll compaction. Such a process can improve the stability of tablet formulations that contain a moisture- and temperature-sensitive drug.

It has been surprisingly found that the use of particular diluents, i.e. a mixture of components (b) and (c), makes direct compression process applicable for the manufacture of olanzapine tablets showing colour stability and dose uniformity.

The term "direct compression" refers to a process, wherein the various components of a tablet are blended, optionally milled, sieved and then compressed into tablets. The blending of the compounds may be achieved in one or more steps. For instance, the active ingredient may first be mixed with a binder and this mixture can than be combined with a mixture of other ingredients. The whole process is preferably performed in the absence of a solvent.

Suitable salts are e.g. disclosed in EP 0454436 B1. If not specified otherwise, all percentages herein are by weight and based on the total weight of the tablet. The active ingredient is evenly distributed in a matrix formed by the other ingredients of the formulation. The tablets do not have a layered structure and, as noted above, are preferably uncoated.

The formulations of the present invention may contain anhydrous forms of olanzapine, which are disclosed e.g. in EP 0733635 B1, therein designated as Form I and Form II; in US 6,348,458, therein designated as Form III, Form IV, Form V; in US 2002/086993 A1, therein designated as form X. Olanzapine used may also be in the polymorphic form A of olanzapine and in the form of olanzapine solvates such as acetonitrile/methylene chloride/water, acetonitrile/water mixed solvates, 2-propanol solvate, methylene chloride solvate IA, methylene chloride solvate IB as disclosed in pending patent application SI 200200175. Also useful are hydrates of olanzapine which are disclosed e.g. in EP 0831098 B1, therein designated as forms B, D, E; in WO 02/18390, therein designated as monohydrate I and dihydrate I. Also useful are solvates disclosed in EP 0733634, therein designated as mono methanol solvate, mono ethanol solvate, mono 1-propanol solvate.

Olanzapine is effective over a wide dosage range, the actual dose administered depending on the condition treated. For example, in the treatment of adult humans, dosages of from 0.25 to 50 mg, preferably 1 to 30 mg, and most preferably 1 to 20 mg per day may be used. A once-daily dosage is normally sufficient, although divided doses may be administered. A preferred tablet of the invention thus comprises 0.25 to 50 mg, preferably 1 to 30 mg and in particular 1 to 20 mg of olanzapine (calculated as the free anhydrous base).

The preferred weight of the tablets is 50 to 1000 mg, most preferably 100 to 500 mg.

The formulations of the invention preferably comprise from 40 to 80 weight %, more preferably from 45 to 75 weight % and most preferably from 50 to 70 weight % of the component (b); 10 to 40 weight %, more preferably from 15 to 30 weight % and most preferably from 15 to 25 weight % of the component (c). According to a preferred embodiment, the tablets of the invention comprise olanzapine or a pharmaceutically acceptable salt thereof as the only pharmaceutically active ingredient.

The component (b) is a monosaccharide, an oligosaccharide or a mixture of a monosaccharide and an oligosaccharide. The terms "monosaccharide" and "oligosaccharide" are intended to also cover derivatives of monosaccharides and oligosaccharides which are the reduced and oxidised forms thereof, such as sugar alcohols, e.g. sorbitol, mannitol, lactitol. Oligosaccharides are compounds comprising 2 to 6, preferably 2 or 3 monosaccharide residues. Carbohydrates comprising more than 6 monosaccharide residues are referred to as polysaccharides.

The component (b) is preferably selected from the group consisting of lactose, sucrose, dextrose, sorbitol, mannitol, lactitol, and mixtures thereof. According to an especially preferred embodiment, the component (b) is lactose, more preferably alpha-lactose and most preferably alpha-lactose monohydrate (Ph. EUR./USP-NF/JP). These compounds may be used in spray-dried form.

The component (c) is a polysaccharide, preferably a polysaccharide comprising from 200 to 10,000 monosaccharide residues, preferably 500 to 10,000 monosaccharide residues, preferably glucose residues. The component (c) is preferably selected from the group consisting of starch, such as pregelatinized starch, cellulose and mixtures thereof.

According to a particularly preferred embodiment, the component (c) is cellulose powder (Ph. Eur.). Although other forms of cellulose may be used, these forms are usually not preferred. Microcrystalline cellulose for instance is relatively hygroscopic, which may adversely affect the stability of the finished product. The same is true for modified starch. Tablets that do not contain microcrystalline cellulose are preferred.

The components (b) and (c) are preferably used in a ratio of 20 to 30 weight %, preferably 25 weight % of the component (c) and 70 to 80 weight %, preferably 75 weight % of the component (b), based on the total weight of the components (b) and (c). Particularly preferred is a mixture of 75 weight % of alpha-lactose monohydrate and 25 weight % cellulose powder (dry matter).

The components (b) and (c) are preferably used in a premixed form, obtained for instance by mixing (b) and (c) and optionally water and spray-drying this mixture. The particle size distribution of the components (b) and (c) or of the premixed form of the components (b) and (c) is preferably as follows: < 100 µm: max. 25 %; < 200 µm: max. 65 %; < 400 µm: min. 98 %, determined by an air jet sieve. For the production of tablets of the present invention it is preferred that the particle size of all excipients is of the same degree.

A premixed form of 75 weight % of alpha-lactose monohydrate and 25 weight % of cellulose powder, which can be used for preparing the tablets of the invention, is sold as Cellactose®80 by Meggle GmbH, Wasserburg, Germany.

In the tablets of the invention the components (b) and (c) serve as diluents. In addition to the components (b) and (c), the tablets may comprise other diluents such as calcium phosphate d.c. grade and povidone (PVP) such as cross-linked PVP.

The main advantages of the combined use of the components (b) and (c) are a high content uniformity due to a low segregation tendency, an excellent compactibility offering possibilities for poorly compressible actives, a stable consistency of the lactose/cellulose system leading to a constant tablet hardness, a good flowability providing a high weight consistency at various tabletting speeds.

The pharmaceutical formulations of the present invention may contain additional ingredients selected from a wide variety of excipients known in the art of pharmaceutical formulation, such as disintegrants, binders, lubricants, and glidants.

Suitable disintegrants are e.g. maize starch, sodium starch glycolate, crospovidone, carboxymethylcellulose sodium, croscarmelose sodium.

Suitable binders are starches, cellulose, PVP. Cellactose has simultaneously the role of a binder and of a diluent and thus the use of an additional binder can be avoided if Cellactose is used in the tablets of the invention. Lubricants and glidants e.g. silica, colloidal anhydrous are used in the formulations of the invention in the usual standard way.

The amounts of excipients used in the formulation are for the diluent from 20 to 90 %, preferably from 50 to 85 weight %, for the disintegrant up to 15 %, preferably up to 10 weight %, for the binder from 5 to 20 %, preferably from 5 to 15 weight %, for the lubricant from 0.25 to 5 %, preferably from 0.5 to 3 weight %, for the glidant from 0.1 to 0.5 %, preferably from 0.2 to 0.5 weight %.

In the preferred embodiment the formulation of the invention comprises 70 to 90 % by weight of a premixed form of components (b) and (c) as defined above, 8 to 12 % by weight of a binder selected from pregelatinized starch, 3 to 10 % by weight of an disintegrant selected from maize starch, 0.3 to 2 % by weight of a lubricant, and 0.2 to 0.4 % by weight of a glidant.

In the state of the art it is stressed that, due to the potent nature of olanzapine, a consistent dose uniformity is imperative and that such uniformity is hardly obtained by direct compression. We have unexpectedly found that, despite the low dose of the active compound in the tablet according to present invention, excellent dose uniformity is obtained, which is shown by analytical results of the content uniformity test where the relative standard deviation (RSD) is from 0.7 to 1 %. This can be attributed to the good flowability of the compression mixture, which therefore provides for a low weight variation, the RSD is equal to or less than 0.8 %, at various compression speeds.

The uniformity of the tablets is determined according to standard procedures as described in Pharmaceutical Dosage Forms, Second Edition, Volume 2, H. A. Lieberman, L. Lachman, J. B. Schwartz (Editors), Marcel Dekker, Inc., New York and Basel, pp. 321 to 325.

The optimisation of tablet formulation is closely related to the intention to have the process of tablet manufacture as simple as possible and to avoid laborious operations that may unnecessarily expose the material to be processed to heat or increased moisture. According to the present invention we avoided the use of solvents for granulation, which may cause conversion to different polymorphic forms or hydrates, and, consequently, avoided moisture conditions under which olanzapine is not stable; additionally, no drying was required during the process, therefore the use of elevated temperatures which may cause discoloration was avoided.

### Examples

### Example 1

### Referential example

| | |
|---|---|
| Olanzapine | 10.00 mg |
| Microcrystalline cellulose | 247.00 mg |
| Starch pregelatinized | 30.00 mg |
| Sodium starch glycolate | 9.00 mg |
| Silica, colloidal anhydrous | 1.00 mg |
| Magnesium stearate | 3.00 mg |

Olanzapine, starch pregelatinized and sodium starch glycolate were mixed together and milled. Microcrystalline cellulose, silica, colloidal anhydrous and magnesium stearate were sieved, blended with the premixture of the drug and compressed into tablets.

Stability results at test conditions:
➢ (1 month, 40 °C/75 % RH, open air): increase in total related compounds from 0.49 % to 3.40 %.

### Example 2

| | |
|---|---|
| Olanzapine | 10.00 mg |
| Cellactose | 247.00 mg |
| Starch pregelatinized | 30.00 mg |
| Sodium starch glycolate | 9.00 mg |
| Silica, colloidal anhydrous | 1.00 mg |
| Magnesium stearate | 3.00 mg |

Olanzapine, starch pregelatinized and sodium starch glycolate were mixed together and milled. Cellactose, silica, colloidal anhydrous and magnesium stearate were sieved and blended with the premixture of the drug. The compression mixture was compressed into tablets.

Stability results at test conditions:
➢ (1 month, 40 °C/75 % RH, open air): increase in total related compounds from 0.40 % to 1.63 %.

### Example 3

| | |
|---|---|
| Olanzapine | 10.00 mg |
| Cellactose | 227.00 mg |
| Starch pregelatinized | 30.00 mg |
| Maize starch | 9.00 mg |
| Silica, colloidal anhydrous | 1.00 mg |
| Magnesium stearate | 3.00 mg |

Olanzapine and maize starch were mixed together and milled. Cellactose, starch pregelatinized and silica, colloidal anhydrous were sieved and blended with the premixture of the drug. Magnesium stearate was sieved and added to the mixture. The compression mixture was compressed into tablets.

Stability results at test conditions:
➢ (6 months, 40 °C/75 % RH, A1/OPA foil): increase in total related compounds from 0.10% to 0.35%.

## Claims

1. A pharmaceutical formulation comprising olanzapine or a pharmaceutically acceptable salt thereof as an active ingredient, obtainable by homogeneously mixing (a) olanzapine or a pharmaceutically acceptable salt thereof with (b) a monosaccharide and/or oligosaccharide and/or a reduced or oxidised form thereof, (c) a polysaccharide and optionally one or more additional excipients, followed by a direct compression of the mixture into tablets in the absence of any solvent.

2. The pharmaceutical formulation of claim 1 comprising 40 to 80 weight % of the component (b).

3. The pharmaceutical formulation of any one of claims 1 to 2 comprising 10 to 40 weight % of the polysaccharide.

4. The pharmaceutical formulation of any one of claims 1 to 3 additionally comprising (d) up to 15 weight % of a disintegrant.

5. The pharmaceutical formulation of any one of claims 1 to 4 additionally comprising (e) 5 to 20 weight % of a binder.

6. The pharmaceutical formulation of any one of claims 1 to 5 additionally comprising (f) 0.25 to 5 weight % of a lubricant.

7. The pharmaceutical formulation of any one of claims 1 to 6 additionally comprising (g) 0.1 to 0.5 weight % of a glidant.

8. The pharmaceutical formulation of any one of claims 1 to 7, wherein the component (b) is selected from the group consisting of lactose, sucrose, dextrose, sorbitol, mannitol, lactitol, and mixtures thereof.

9. The pharmaceutical formulation of claim 8, wherein the component (b) is lactose.

10. The pharmaceutical formulation of any one of claims 1 to 9, wherein the polysaccharide is selected from the group consisting of starch, cellulose, and mixtures thereof.

11. The pharmaceutical formulation of claim 10, wherein the polysaccharide is cellulose.

12. The pharmaceutical formulation of claim 11, wherein a mixture of 20 to 30 weight % of cellulose and 70 to 80 weight % of lactose is used as the components (b) and (c).

13. The pharmaceutical formulation of claim 12 comprising
70 to 90 weight % of a mixture of 20 to 30 weight % of cellulose and 70 to 80 weight % of lactose;
8 to 12 weight % of a binder;
3 to 10 weight % of a disintegrant;
0.3 to 2 weight % of a lubricant; and
0.2 to 0.4 weight % of a glidant.

14. The pharmaceutical formulation of any one of claims 1 to 13 comprising olanzapine as the only pharmaceutically active ingredient.

15. The pharmaceutical formulation of any one of claims 1 to 14 having the form of an uncoated tablet

16. A process for preparing a stable pharmaceutically solid oral formulation according to any one of claims 1 to 15 comprising combining (a) olanzapine or a pharmaceutically acceptable salt thereof with (b) a monosaccharide and/or oligosaccharide and/or a reduced or oxidised form thereof, (c) a polysaccharide and optionally one or more of components (d) to (g), followed by a direct compression of the mixture into tablets in the absence of any solvent

## Patentansprüche

1. Pharmazeutische Formulierung, die Olanzapin oder ein pharmazeutisch annehmbares Salz davon als wirksamen Bestandteil enthält, **dadurch** erhältlich, dass (a) Olanzapin oder ein pharmazeutisch annehmbares Salz davon mit (b) einem Monosaccharid und/oder Oligosaccharid und/oder einer reduzierten oder oxidierten Form davon, (c) einem Polysaccharid und gegebenenfalls einem oder mehreren zusätzlichen Hilfsstoffen homogen gemischt wird, gefolgt von direktem Verpressen der Mischung zu Tabletten in Abwesenheit von Lösungsmittel.

2. Pharmazeutische Formulierung nach Anspruch 1, die 40 bis 80 Gew.-% der Komponente (b) enthält.

3. Pharmazeutische Formulierung nach einem der Ansprüche 1 bis 2, die 10 bis 40 Gew.-% Polysaccharid enthält.

4. Pharmazeutische Formulierung nach einem der Ansprüche 1 bis 3, die zusätzlich (d) bis zu 15 Gew.-% Sprengmittel enthält.

5. Pharmazeutische Formulierung nach einem der Ansprüche 1 bis 4, die zusätzlich (e) 5 bis 20 Gew.-% Bindemittel enthält.

6. Pharmazeutische Formulierung nach einem der Ansprüche 1 bis 5, die zusätzlich (f) 0,25 bis 5 Gew.-% Schmiermittel enthält.

7. Pharmazeutische Formulierung nach einem der Ansprüche 1 bis 6, die zusätzlich (g) 0,1 bis 0,5 Gew.-% Gleitmittel enthält.

8. Pharmazeutische Formulierung nach einem der - Ansprüche 1 bis 7, bei der die Komponente (b) ausgewählt ist aus der Gruppe bestehend aus Lactose, Sucrose, Dextrose, Sorbitol, Mannitol, Läctitol und Mischungen davon.

9. Pharmazeutische Formulierung nach Anspruch 8, bei der die Komponente (b) Lactose ist.

10. Pharmazeutische Formulierung nach einem der Ansprüche 1 bis 9, bei der das Polysaccharid ausgewählt ist aus der Gruppe bestehend aus Stärke, Cellulose und Mischungen davon.

11. Pharmazeutische Formulierung nach Anspruch 10, bei der das Polysaccharid Cellulose ist.

12. Pharmazeutische Formulierung nach Anspruch 11, bei der eine Mischung von 20 bis 30 Gew.-% Cellulose und 70 bis 80 Gew.-% Lactose als Komponenten (b) and (c) verwendet wird.

13. Pharmazeutische Formulierung nach Anspruch 12, die
70 bis 90 Gew.-% einer Mischung von 20 bis 30 Gew.-% Cellulose und 70 bis 80 Gew.-% Lactose,
8 bis 12 Gew.-% Bindemittel,
3 bis 10 Gew.-% Sprengmittel,
0,3 bis 2 Gew.-% Schmiermittel und
0,2 bis 0,4 Gew.-% Gleitmittel enthält.

14. Pharmazeutische Formulierung nach einem der Ansprüche 1 bis 13, die Olanzapin als einzigen pharmazeutisch wirksamen Bestandteil enthält.

15. Pharmazeutische Formulierung nach einem der Ansprüche 1 bis 14, die die Form einer unbeschichteten Tablette aufweist.

16. Verfahren zur Herstellung einer stabilen pharmazeutischen festen oralen Formulierung gemäß einem der Ansprüche 1 bis 15, bei dem man (a) Olanzapin oder ein pharmazeutisch annehmbares Salz davon mit (b) einem Monosaccharid und/oder Oligosaccharid und/oder einer reduzierten oder oxidierten Form davon, (c) einem Polysaccharid und gegebenenfalls einer oder mehreren der Komponenten (d) bis (g) kombiniert, und man dann die Mischung in Abwesenheit von Lösungsmittel direkt zu Tabletten verpresst.

## Revendications

1. Formulation pharmaceutique comprenant de l'olanzapine ou un sel pharmaceutiquement acceptable de celle-ci en tant que principe actif, pouvant être obtenue en mélangeant de manière homogène (a) de l'olanzapine ou un sel pharmaceutiquement acceptable de celle-ci avec (b) un monosaccharide et/ou un oligosaccharide et/ou une forme réduite ou oxydée de ceux-ci, (c) un polysaccharide et éventuellement un ou plusieurs excipients supplémentaires, ceci suivi d'une compression directe du mélange en comprimés en l'absence de tout solvant.

2. Formulation pharmaceutique selon la revendication 1, comprenant 40 à 80 % en poids du composant (b).

3. Formulation pharmaceutique selon l'une quelconque des revendications 1 et 2, comprenant 10 à 40 % en poids du polysaccharide.

4. Formulation pharmaceutique selon l'une quelconque des revendications 1 à 3, comprenant en outre (d) jusqu'à 15 % en poids d'un délitant.

5. Formulation pharmaceutique selon l'une quelconque des revendications 1 à 4, comprenant en outre (e) 5 à 20 % en poids d'un liant.

6. Formulation pharmaceutique selon l'une quelconque des revendications 1 à 5, comprenant en outre (f) 0,25 à 5 % en poids d'un lubrifiant.

7. Formulation pharmaceutique selon l'une quelconque des revendications 1 à 6, comprenant en outre (g) 0,1 à 0,5 % en poids d'un glissant.

8. Formulation pharmaceutique selon l'une quelconque des revendications 1 à 7, dans laquelle le composant (b) est choisi dans le groupe constitué de lactose, saccharose, dextrose, sorbitol, mannitol, lactitol et de mélanges de ceux-ci.

9. Formulation pharmaceutique selon la revendication 8, dans laquelle le composant (b) est du lactose.

10. Formulation pharmaceutique selon l'une quelconque des revendications 1 à 9, dans laquelle le polysaccharide est choisi dans le groupe constitué d'amidon, cellulose et de mélanges de ceux-ci.

11. Formulation pharmaceutique selon la revendication 10, dans laquelle le polysaccharide est de la cellulose.

12. Formulation pharmaceutique selon la revendication 11, dans laquelle un mélange de 20 à 30 % en poids de cellulose et de 70 à 80 % en poids de lactose est utilisé en tant que composants (b) et (c).

13. Formulation pharmaceutique selon la revendication 12, comprenant
70 à 90 % en poids d'un mélange de 20 à 30 % en poids de cellulose et de 70 à 80 % en poids de lactose ;
8 à 12 % en poids d'un liant ;
3 à 10 % en poids d'un délitant ;
0,3 à 2 % d'un lubrifiant ; et
0,2 à 0,4 % en poids d'un glissant.

14. Formulation pharmaceutique selon l'une quelconque des revendications 1 à 13, comprenant de l'olanzapine en tant que seul composant pharmaceutiquement actif.

15. Formulation pharmaceutique selon l'une quelconque des revendications 1 à 14, ayant la forme d'un comprimé non enrobé.

16. Procédé de préparation d'une formulation orale solide pharmaceutiquement stable selon l'une quelconque des revendications 1 à 15, comprenant la combinaison (a) d'olanzapine ou d'un sel pharmaceutiquement acceptable de celle-ci avec (b) un monosaccharide et/ou un oligosaccharide et/ou une forme réduite ou oxydée de ceux-ci, (c) un polysaccharide et éventuellement un ou plusieurs des composants (d) à (g), suivie d'une compression directe du mélange en comprimés en l'absence de tout solvant.
